# EUROPEAN PATENT SPECIFICATION

(11) **EP 4 013 463 B1**
(45) Date of publication and mention of the grant of the patent: **26.06.2024**
(21) Application number: 20768414.3
(22) Date of filing: 12.08.2020
(51) Int. Cl.: A61L 27/14, A61L 27/38, A61L 27/56, A61L 27/16, A61L 27/18, A61L 27/20, A61L 27/22, A61L 27/34, A61L 27/50, A61L 27/52

(54) **GINGIVAL GRAFT**
ZAHNFLEISCHTRANSPLANTAT
GREFFE GINGIVALE

(30) Priority: 12.08.2019 US 201962885345 P; 01.04.2020 US 202063003319 P
(43) Date of publication of application: 22.06.2022
(73) Proprietor: Corneat Vision Ltd., 4366411 Ra'anana (IL)
(72) Inventor: LITVIN, Gilad, 4493500 Moshav Sde Varburg (IL); ALEY-RAZ, Almog, 4493500 Moshav Sde Warburg (IL); ASSOULINE, Patricia, Kafar Yona, 4037012 (IL)
(74) Representative: Pearl Cohen Zedek Latzer Baratz UK LLP
(86) International application number: PCT/IL2020/050879
(87) International publication number: WO 2021/028912

(56) References cited:
- WO-A1-90/11730
- WO-A1-2016/049682
- WO-A2-2013/094992
- WENLU JIANG ET AL: "Incorporation of aligned PCL-PEG nanofibers into porous chitosan scaffolds improved the orientation of collagen fibers in regenerated periodontium", ACTA BIOMATERIALIA, vol. 25, 1 October 2015 (2015-10-01), pages 240-252, XP055753583, AMSTERDAM, NL ISSN: 1742-7061, DOI: 10.1016/j.actbio.2015.07.023
- GHOGHA LOTFI ET AL: "A Clinical and Histologic Evaluation of Gingival Fibroblasts Seeding on a Chitosan-Based Scaffold and Its Effect on the Width of Keratinized Gingiva in Dogs", JOURNAL OF PERIODONTOLOGY., vol. 82, no. 9, 1 September 2011 (2011-09-01), pages 1367-1375, XP055754084, US ISSN: 0022-3492, DOI: 10.1902/jop.2011.100604
- Ito K ET AL: "A preliminary comparative study of the guided tissue regeneration and free gingival graft procedures for adjacent facial root coverage", Quintessence international (Berlin, Germany : 1985), 1 May 2000 (2000-05-01), pages 319-326, XP055841403, Germany Retrieved from the Internet: URL:http://www.quintpub.com/userhome/qi/qi _31_5_ito_4.pdf [retrieved on 2021-09-15]

## Description

### BACKGROUND OF THE INVENTION

Gingival recession, also known as receding gums, is a condition that is manifested by exposure of the roots of the teeth caused by a loss of gum tissue and/or retraction of the gingival margin from the crown of the teeth first down the neck exposing it, then eventually exposing parts of the root. Gum recession is a common typical condition in adults over the age of 40, but it may also occur starting from the age of a teenager, or around the age of 10. It may exist with or without concomitant decrease in crown-to-root ratio (recession of alveolar bone). In most cases, receding gums are a progressive condition that occurs gradually over years. Classification of the condition is based on the position of interdental papilla and buccal/lingual/palatal recessions.

There are many possible causes for gingival recession: (i) the most common cause is gum disease (periodontal disease); (ii) overaggressive brushing and improper flossing (i.e., flossing too roughly or aggressively) which may cut into the gums; (iii) hereditary thin, fragile or insufficient gingival tissue predisposes to gingival recession; (iv) consumption of tobacco products, which affect the mucous membrane lining in the mouth; (v) self-inflicted trauma, such as habits like digging a fingernail or pencil into the gum and piercings in the lip or tongue; (vi) diseases such as: scurvy (lack of dietary vitamin C), acute necrotizing ulcerative gingivitis; (vii) abnormal tooth position, such as tooth crowding, giving inadequate cover of one or more teeth by the jaw bone; (viii) intentional gingival retraction performed during dental surgery procedures.

The following signs and symptoms may indicate gum recession: tooth mobility, dentin hypersensitivity, changes in the teeth appearance (appear longer, roots are exposed and visible, tooth feels notched at the gum line, change in the tooth color, spaces between teeth), cavities below the gum line. If the gum recession is caused by gingivitis, the following symptoms may also be present: puffy, red, or swollen (inflamed) gums, gum bleeding while brushing or flossing, bad breath (halitosis). In some cases, it is the treatment of gingivitis that reveals a gum recession problem, that was previously masked by the gums swelling.

The solutions for gingival recession conditions depend on the shape of the gum recession and the levels of bone around the teeth. Such solutions include regeneration of areas of gum recession with new gum tissue using a variety of gum grafting procedures. These procedures involve repositioning of adjacent gum tissue to cover the recession (called a pedicle graft) or use of a free graft of gingiva with or without a connective tissue graft harvested from the roof of the mouth (called collectively a free gingival graft or a Subepithelial connective tissue graft). Alternatively, a material called acellular dermal matrix (processed donated human skin allograft) may be used as a replacement to connective tissue coming from the patient's own palate.

Recent advances have seen the introduction of platelet derived growth factor (PDGF) infused bone graft material. This material is usually combined with the cellular matrix to form a soft bone paste that is then covered by the allograft. The development of this type of bone and tissue cellular matrix (also known as ortho filler) results in greater osseointegration with the patient's healthy bone and soft tissue. Healing from such procedures requires 2-4 weeks. After a few months the results are evaluated, and, in some cases, the new tissue needs to be reshaped in a further procedure to get an optimal result.

The solutions available for gingival recession have many drawbacks. These solutions require at least two painful, technically demanding and time-consuming dental operations, and are limited by the potential graft's thickness and length. Current solutions, being tissue based, are bio-degradable and according to current professional literature have limited long-term efficiency and stability.

WO 2013/094992 discloses the use of bone morphogenetic proteins for regenerating periodontal tissue comprising a periodontal ligament and cementum of an alveolar bone and a tooth and regenerating the pulp tissue of an extracted tooth using a growth factor in replantation of the extracted tooth. Jiang et al. (Acta Biomaterialia, 2015, vol. 25, pages 240-252, XP055753583, DOI: 10.1016/j.actbio.2015.07.023) disclose the incorporation of aligned PCL-PEG nanofibers into porous chitosan scaffolds improving the orientation of collagen fibers in regenerated periodontium. Lotfi et al. (Journal of Periodontology, 2011, vol. 82, no. 9, pages 1367-1375, XP055754084, DOI: 10.1902/jop.2011.100604) describe a clinical and histologic evaluation of gingival fibroblasts seeding on a chitosan-based scaffold and its effect on the width of keratinized gingiva in dogs. Ito et al. (Quintessence International, Berlin, Germany: 1985, 2000, pages 319-326, XP055841403) describes a preliminary comparative study of guided tissue regeneration and free gingival graft procedures for adjacent facial root coverage.

### SUMMARY OF THE INVENTION

Thus, there is a need for providing an effective and stable solution for gingival recession that is on the one hand versatile, making the procedure simple to perform, and on the other hand bio-stable, having an improved and lasting effect.

The present invention provides a synthetic gingival patch graft having a porous polymeric structure with pores of less than 5 µm wherein said patch is a non-degradable patch and wherein said synthetic gingival patch is a tissue replacement patch. The invention also provides a synthetic gingival patch graft having a porous polymeric structure with pores of between about 0.01 µm to about 5 µm.

When referring to a *"synthetic gingival patch graft"* it should be understood to relate to an implant of synthetic polymeric material used to repair a defect the gingival tissue of a subject. In some embodiments, said patch graft of the invention is in the form of a sheet.

In some embodiments, said synthetic gingival patch graft having a porous polymeric structure, has pores of between about 0.01 µm (microns) to 5 µm (microns). In some embodiments a synthetic gingival patch graft having a porous polymeric structure, has pores of 0.01, 0.05, 0.1, 0.5, 1, 1.5, 2, 2.5, 3, 3.5, 4, 4.5, 5 µm.

In some embodiments, said synthetic gingival patch graft of the invention is a biocompatible patch.

In some embodiments, said synthetic gingival patch graft of the invention has a thickness of between 100 - 1000 µm. In other embodiments, said synthetic gingival patch graft of the invention has a thickness of between 10 - 100 µm. In other embodiments, said synthetic gingival patch graft of the invention has a thickness of between 1000 - 2500 µm.

In some embodiments, said porous polymeric structure comprises at least one polymer. In other embodiments, said porous polymeric structure comprises nanofibers (in some embodiments, said nanofibers are 500 nm to a few micrometers in thickness).

In some embodiments, said porous polymeric structure comprises at least one porous electrospun polymer. In some other embodiments said porous polymeric structure comprises at least one porous printed polymer (using for example a 3D printing device).

In some further embodiments, said porous polymeric structure comprises at least one polymer selected from polycarbonate, poly(DTE carbonate), polycaprolactone (PCL), Poly(DL-lactide-co-caprolactone, Poly(ethylene-co-vinyl acetate) vinyl acetate, Poly(methyl methacrylate), Poly(propylene carbonate), Poly(vinylidene fluoride), Polyacrylonitrile, Polycarbomethylsilane, Polystyrene, Polyvinylpyrrolidone, poly vinyl alcohol (PVA), polyethylene oxide (PEO), polyurethane (including aromatic polyurethane), polyvinyl chloride (PVC), chitosan, alginate, polyhydroxybutyrate and its copolymers, Nylon 11, Cellulose acetate, poly(3-hydroxybutyric acid-co-3-hydroxyvaleric acid), poly(DL-lactide), and poly(L-lactide) or any combination thereof.

Electrospun fibers are typically several orders in magnitude smaller than those produced using conventional spinning techniques. By optimizing parameters such as: i) the intrinsic properties of the solution including the polarity and surface tension of the solvent, the molecular weight and conformation of the polymer chain, and the viscosity, elasticity, and electrical conductivity of the solution; and ii) the operational conditions such as the strength of electric field, the distance between spinneret and collector, and the feeding rate of the solution, electrospinning is capable of generating fibers as thin as tens of nanometers in diameter. Additional parameters that affect the properties of electrospun fiber include the molecular weight, molecular-weight distribution and structure (branched, linear etc.) of the polymer, solution properties (viscosity, conductivity and surface tension), electric potential, flow rate and concentration, distance between the capillary and collection screen, ambient parameters (temperature, humidity and air velocity in the chamber), motion of target screen (collector) and so forth. Fabrication of highly porous fibers may be achieved by electrospinning the jet directly into a cryogenic liquid. Well-defined pores developed on the surface of each fiber as a result of temperature-induced phase separation between the polymer and the solvent and the evaporation of solvent under a freeze-drying condition.

Several approaches have been developed to organize electrospun fibers into aligned arrays. For example, electrospun fibers can be aligned into a uniaxial array by replacing the single-piece collector with a pair of conductive substrates separated by a void gap. In this case, the nanofibers tend to be stretched across the gap oriented perpendicular to the edges of the electrodes. It was also shown that the paired electrodes could be patterned on an insulating substrate such as quartz or polystyrene so the uniaxially aligned fibers could be stacked layer-by-layer into a 3D lattice. By controlling the electrode pattern and/or the sequence for applying high voltage, it is also possible to generate more complex architectures consisting of well-aligned nanofibers.

Electrospun nanofibers could also be directly deposited on various objects to obtain nanofiber-based constructs with well-defined and controllable shapes. In addition, one can manually process membranes of aligned or randomly oriented nanofibers into various types of constructs after electrospinning: for example, fabrication of a tube by rolling up a fibrous membrane or the preparation of discs with controllable diameters by punching a fibrous membrane.

The present invention relates to any electrospinning technique known in the art, which includes Electrospinning, J. Stanger, N. Tucker, and M. Staiger, I-Smithers Rapra publishing (UK), An Introduction to Electrospinning and Nanofibers, S. Ramakrishna , K. Fujihara, W-E Teo, World Scientific Publishing Co. Pte Ltd (Jun 2005), Electrospinning of micro- and nanofibers: fundamentals and applications in separation and filtration processes, Y. Fillatov, A. Budyka, and V. Kirichenko (Trans. D. Letterman), Begell House Inc., New York, USA, 2007.

Suitable electrospinning techniques are disclosed, e.g., in International Patent Application, Publication Nos. WO 2002/049535, WO 2002/049536, WO 2002/049536, WO 2002/049678, WO 2002/074189, WO 2002/074190, WO 2002/074191, WO 2005/032400 and WO 2005/065578. It is to be understood that although the according to the presently preferred embodiment of the invention is described with a particular emphasis to the electrospinning technique, it is not intended to limit the scope of the invention to the electrospinning technique. Representative examples of other spinning techniques suitable for the present embodiments include a wet spinning technique, a dry spinning technique, a gel spinning technique, a dispersion spinning technique, a reaction spinning technique or a tack spinning technique. Such and other spinning techniques are known in the art and disclosed, e.g., in U.S. Patent Nos., 3,737,508, 3,950,478, 3,996,321, 4,189,336, 4,402,900, 4,421,707, 4,431,602, 4,557,732, 4,643,657, 4,804,511, 5,002,474, 5,122,329, 5,387,387, 5,667,743, 6,248,273 and 6,252,031.

In some embodiments, said synthetic gingival patch graft of the invention further comprises at least one active agent. In some embodiments, said at least one active agent is selected from a protein, type I collagen, fibronectin, or TGF- beta 2, heparin, growth factors, antibodies, antimetabolites, chemotherapeutic agents, anti-inflammatory agent, anti-biotic agent, and any combinations thereof.

In some embodiments, said synthetic gingival patch graft of the invention is cut into a designated shape (in some embodiments, said cut is laser cut, manual cut, pressure cut and so forth).

In some embodiments, said synthetic gingival patch graft of the invention further comprises at least one non-porous layer. In some embodiments, said at least one non-porous layer is in the form of a film. When used as a tissue replacement in periodontal surgery, said at least one non-porous layer or film is placed on the bone side of the cavity to be filled.

When referring to a *"non-porous layer"* it should be understood to encompass a film layer that has substantially no pores, thus not penetrable, impervious as compared with said porous layer of the gingival graft patch of the invention.

In some embodiments, said non-porous layer is a biocompatible patch.

In some embodiments, said non-porous layer has a thickness of between 100 - 1000 µm. In other embodiments, said non-porous layer has a thickness of between 10 - 100 µm. In other embodiments, said non-porous layer has a thickness of between 1000 - 2500 µm.

In some embodiments, said non-porous polymeric structure comprises at least one polymer. In other embodiments, said non-porous polymeric structure comprises nanofibers (in some embodiments, said nanofibers are 500 nm to a few micrometers in thickness).

In some further embodiments, said non-porous polymeric structure comprises at least one polymer selected from polycarbonate, poly(DTE carbonate), polycaprolactone (PCL), Poly(DL-lactide-co-caprolactone, Poly(ethylene-co-vinyl acetate) vinyl acetate, Poly(methyl methacrylate), Poly(propylene carbonate), Poly(vinylidene fluoride), Polyacrylonitrile, Polycarbomethylsilane, Polystyrene, Polyvinylpyrrolidone, poly vinyl alcohol (PVA), polyethylene oxide (PEO), polyurethane (including aromatic polyurethane), polyvinyl chloride (PVC), chitosan, alginate, polyhydroxybutyrate and its copolymers, Nylon 11, Cellulose acetate, poly(3-hydroxybutyric acid-co-3-hydroxyvaleric acid), poly(DL-lactide), and poly(L-lactide) or any combination thereof.

When relating to *"periodontal disease, condition or symptom"* it should be understood to encompass inflammatory conditions affecting the tissues surrounding the teeth such as for example gum disease, gingivitis, periodontitis, tooth decay, tooth loss, bone loss and any combinations thereof.

In some embodiments, such periodontal disease, condition or symptom may cause the need for periodontal surgery.

The term *"periodontal surgery"* is meant to encompass a form of dental surgery that prevents or corrects anatomical, traumatic, developmental, or plaque-induced defects in the bone, gingiva, or alveolar mucosa. The objectives of this surgery include accessibility of instruments to root surface, elimination of inflammation, creation of an oral environment for plaque control, periodontal diseases control, oral hygiene maintenance, maintain proper embrasure space, address gingiva-alveolar mucosa problems, and esthetic improvement. The surgical procedures include among others, crown lengthening, frenectomy, mucogingival flap surgery, gingivectomy, apically repositioned flap (APF) surgery, apically repositioned flap (APF) with osseous reduction (osteoplasty/ostectomy) and any combinations thereof.

The invention further provides a synthetic gingival patch graft as disclosed herein above and below for use in periodontal and/ or dental surgery.

The invention further provides a synthetic gingival patch graft as disclosed herein above and below for use in the treatment of periodontal disease, condition or symptom.

The invention further provides a synthetic gingival patch graft as disclosed herein above and below for use in the treatment of gingival recession.

The invention further provides a synthetic gingival patch graft as disclosed herein above and below for use in periodontal root coverage procedure.

The invention further provides a synthetic gingival patch graft as disclosed herein above and below for use in the treatment of periodontal root disease, condition or symptom.

In some embodiments, a synthetic gingival patch graft of the invention is a periodontal tissue replacement patch.

The invention further provides a device comprising at least one synthetic gingival patch graft of the invention. In some embodiments said device is a dental implant comprising said at least one synthetic gingival patch graft of the invention (for example, covered by said at least one synthetic gingival patch graft of the invention).

The invention further provides a kit comprising at least one synthetic gingival patch graft of the invention, means for its periodontal implanting/placement in the gingival tissue of a subject and instructions for use.

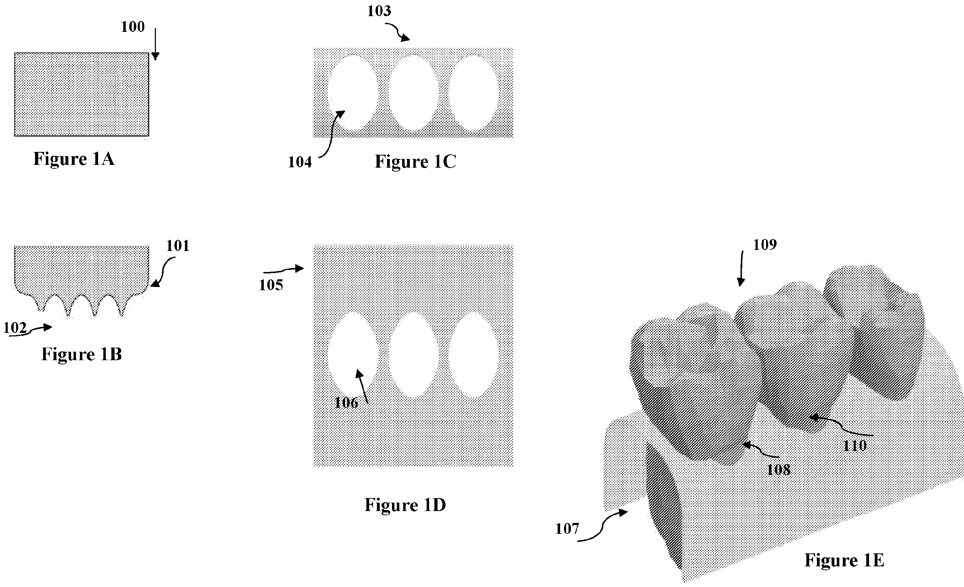

In some embodiments, instructions of use may include: instructions for the care giver how to custom cut the graft patch of the invention, how to place said graft patch invention for example, over the teeth and on the gingival tissue (see for example Figure 1, 110).

### BRIEF DESCRIPTION OF THE DRAWINGS

The subject matter regarded as the invention is particularly pointed out and distinctly claimed in the concluding portion of the specification. The invention, however, both as to organization and uses thereof, together with objects, features, and advantages thereof, may best be understood by reference to the following detailed description when read with the accompanying drawings in which:
**Figs. 1A-1E** show embodiments of a synthetic gingival patch graft of the invention.
**Figs 2A-2B** show embodiments of a synthetic gingival patch graft of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

Figure 1A shows a synthetic gingival patch graft of the invention (100). Figure 1B shows the synthetic gingival patch graft of the invention (101) with grooves (102) cut in a predetermined shape to be placed periodontally. Figure 1C and Figure 1D show embodiments of a synthetic gingival patch graft of the invention (103) and (105) with holes (104) and (106) cut in a predetermined shape to be placed periodontally. Figure 1E shows a synthetic gingival patch graft of the invention (107), placed on a representative periodontal segment (109), wherein the holes (108) cut into the patch allow the placement over the teeth and on the gingival tissue (110).

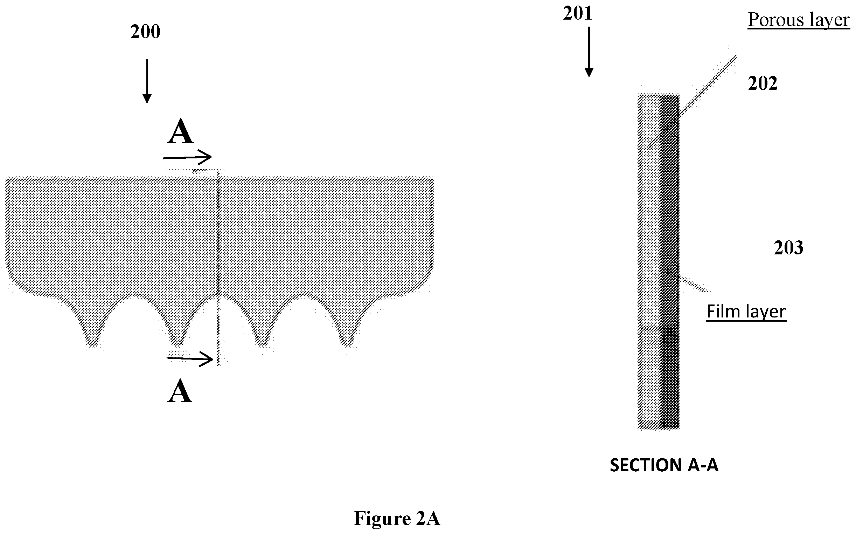

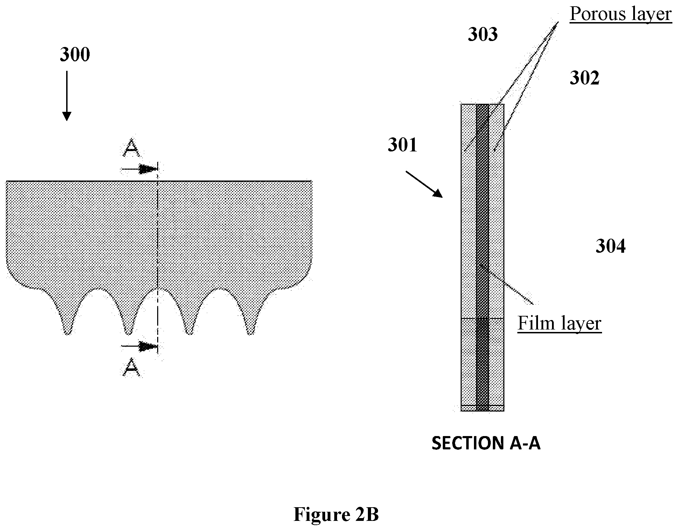

Figure 2A shows a synthetic gingival patch graft of the invention (200 and 201 the AA cross section of 200) that comprises in addition to the porous layer (202), a non-porous (film) layer (203). Figure 2B shows synthetic gingival patch graft of the invention (300 and 301 the AA cross section of 300) that comprises two porous layers (302 and 303) and one non-porous (film) layer (304) in between them.

## Claims

1. A synthetic gingival patch graft having a porous polymeric structure with pores of less than 5 µm, wherein said patch is a non-degradable patch, wherein said synthetic gingival patch is a tissue replacement patch.

2. A synthetic gingival patch graft having a porous polymeric structure with pores of between about 0.01 µm to about 5 µm.

3. A synthetic gingival patch graft according to claims 1 or 2, being a biocompatible patch.

4. A synthetic gingival patch graft according to any one of the preceding claims, having a thickness of between 10 - 100µm.

5. A synthetic gingival patch graft according to any one of the preceding claims, wherein said porous polymeric structure comprises at least one polymer.

6. A synthetic gingival patch graft according to any one of the preceding claims, wherein said porous polymeric structure comprises nanofibers.

7. A synthetic gingival patch graft according to any one of the preceding claims, wherein said porous polymeric structure comprises at least one porous electrospun polymer.

8. A synthetic gingival patch graft according to any one of the preceding claims, further comprising at least one active agent.

9. A synthetic gingival patch graft according to any one of the preceding claims further comprising at least one non-porous layer.

10. A synthetic gingival patch graft according to any one of the preceding claims for use in the treatment of periodontal disease, condition or symptom.

11. A synthetic gingival patch graft according to any one of the preceding claims for use in the treatment of gingival recession.

12. A synthetic gingival patch graft according to any one of the preceding claims for use in the treatment of periodontal root disease, condition or symptom.

13. A device comprising at least one synthetic gingival patch graft as defined in any one of the preceding claims.

14. A kit comprising at least one synthetic gingival patch graft as defined in any one of the preceding claims, means for its implanting/placement in the gingival tissue of a subject and instructions for use.

## Patentansprüche

1. Synthetisches Gingiva-Patch-Transplantat, das eine poröse Polymerstruktur mit Poren von weniger als 5 µm Größe hat, wobei das synthetische Patch ein nicht-abbaubares Patch ist, wobei das synthetische Gingiva-Patch ein Gewebsersatz-Patch ist.

2. Synthetisches Gingiva-Patch-Transplantat, das eine poröse Polymerstruktur mit Poren von zwischen etwa 0,01 µm und 5 µm Größe hat.

3. Synthetisches Gingiva-Patch-Transplantat nach einem der Ansprüche 1 oder 2, wobei das Patch ein biokompatibles Patch ist.

4. Synthetisches Gingiva-Patch-Transplantat nach einem der vorhergehenden Ansprüche, das eine Dicke von 10-100 µm hat.

5. Synthetisches Gingiva-Patch-Transplantat nach einem der vorhergehenden Ansprüche, wobei die poröse Polymerstruktur mindestens ein Polymer umfasst.

6. Synthetisches Gingiva-Patch-Transplantat nach einem der vorhergehenden Ansprüche, wobei die poröse Polymerstruktur Nanofasern umfasst.

7. Synthetisches Gingiva-Patch-Transplantat nach einem der vorhergehenden Ansprüche, wobei die poröse Polymerstruktur mindestens ein elektrogesponnenes Polymer umfasst.

8. Synthetisches Gingiva-Patch-Transplantat nach einem der vorhergehenden Ansprüche ferner umfassend mindestens ein aktives Agens.

9. Synthetisches Gingiva-Patch-Transplantat nach einem der vorhergehenden Ansprüche ferner umfassend mindestens eine nicht-poröse Schicht.

10. Synthetisches Gingiva-Patch-Transplantat nach einem der vorhergehenden Ansprüche zur Anwendung bei Parodontose, einem parodontalen Zustand oder Symptom.

11. Synthetisches Gingiva-Patch-Transplantat nach einem der vorhergehenden Ansprüche zur Anwendung bei der Behandlung von Zahnfleischrückgang.

12. Synthetisches Gingiva-Patch-Transplantat nach einem der vorhergehenden Ansprüche zur Anwendung bei der Behandlung einer parodontalen Wurzelerkrankung, eines parodontalen Zustands oder Symptoms.

13. Vorrichtung umfassend mindestens ein synthetisches Gingiva-Patch-Transplantat entsprechend der Definition in einem der vorhergehenden Ansprüche.

14. Bestecksatz umfassend mindestens ein synthetisches Gingiva-Patch-Transplantat entsprechend der Definition in einem der vorhergehenden Ansprüche, Mittel zum Implantieren/Platzieren desselben im Zahnfleischgewebe eines Subjekts und Anwendungsanleitungen.

## Revendications

1. Greffe de patch gingival synthétique ayant une structure polymère poreuse avec des pores de moins de 5 µm, dans laquelle ledit patch est un patch non dégradable, ledit patch gingival synthétique étant un patch de remplacement de tissu.

2. Greffe de patch gingival synthétique ayant une structure polymère poreuse avec des pores compris entre environ 0,01 µm et environ 5 µm.

3. Greffe de patch gingival synthétique selon les revendications 1 ou 2, étant un patch biocompatible.

4. Greffe de patch gingival synthétique selon l'une quelconque des revendications précédentes, ayant une épaisseur comprise entre 10 et 100 µm.

5. Greffe de patch gingival synthétique selon l'une quelconque des revendications précédentes, dans laquelle ladite structure polymère poreuse comprend au moins un polymère.

6. Greffe de patch gingival synthétique selon l'une quelconque des revendications précédentes, dans laquelle ladite structure polymère poreuse comprend des nanofibres.

7. Greffe de patch gingival synthétique selon l'une quelconque des revendications précédentes, dans laquelle ladite structure polymère poreuse comprend au moins un polymère poreux électrofilé.

8. Greffe de patch gingival synthétique selon l'une quelconque des revendications précédentes, comprenant en outre au moins un agent actif.

9. Greffe de patch gingival synthétique selon l'une quelconque des revendications précédentes comprenant en outre au moins une couche non poreuse.

10. Greffe de patch gingival synthétique selon l'une quelconque des revendications précédentes destinée à être utilisée dans le traitement d'une maladie, d'un état ou d'un symptôme parodontal.

11. Greffe de patch gingival synthétique selon l'une quelconque des revendications précédentes destinée à être utilisée dans le traitement de la récession gingivale.

12. Greffe de patch gingival synthétique selon l'une quelconque des revendications précédentes destinée à être utilisée dans le traitement d'une maladie, d'un état ou d'un symptôme parodontal.

13. Dispositif comprenant au moins une greffe de patch gingival synthétique telle que définie dans l'une quelconque des revendications précédentes.

14. Kit comprenant au moins une greffe de patch gingival synthétique telle que définie dans l'une quelconque des revendications précédentes, des moyens pour son implantation/placement dans le tissu gingival d'un sujet et des instructions d'utilisation.
